(19) Europäisches Patentamt
European Patent Office
Office européen des brevets

(11) **EP 4 018 998 A1**

(12) **EUROPEAN PATENT APPLICATION**
published in accordance with Art. 153(4) EPC

(43) Date of publication:
**29.06.2022 Bulletin 2022/26**

(21) Application number: **20853956.9**

(22) Date of filing: **19.08.2020**

(51) International Patent Classification (IPC):
*A61K 8/73* (2006.01)  *A61K 8/04* (2006.01)
*A61Q 19/00* (2006.01)

(52) Cooperative Patent Classification (CPC):
**A61K 8/04; A61K 8/73; A61Q 19/00**

(86) International application number:
**PCT/JP2020/031318**

(87) International publication number:
**WO 2021/033725 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2019 JP 2019151229**

(71) Applicant: **Shiseido Company, Ltd.**
**Chuo-ku**
**Tokyo 104-0061 (JP)**

(72) Inventors:
• OKA, Takashi
  Tokyo 104-0061 (JP)
• SHIMIZU, Hiroko
  Tokyo 104-0061 (JP)
• YOSHIMURA, Mika
  Tokyo 104-0061 (JP)

(74) Representative: **Cabinet Beau de Loménie**
**158, rue de l'Université**
**75340 Paris Cedex 07 (FR)**

(54) **COSMETIC**

(57) There is provided a cosmetic comprising a hyaluronic acid particle, which can maintain a non-invasively excellent moisture retaining property over a long period of time.

A cosmetic of the present disclosure comprises an aqueous medium, and a hyaluronic acid particle dispersed in the aqueous medium, wherein the average particle diameter of the hyaluronic acid particle is 200nm or less.

FIG. 1

EP 4 018 998 A1

## Description

FIELD

The present disclosure relates to a cosmetic for moisturizing.

BACKGROUND

In order to moisturize the skin, hyaluronic acid having a moisturizing function has been utilized in the field of cosmetics and the like.

Patent Literature 1 discloses a cosmetic comprising a hyaluronic acid-supported nanoparticle in which hyaluronic acid is supported on at least one of an inside or a surface of a nanoparticle formed of either polylactic acid, polyglycolic acid, and a lactic acid-glycolic acid copolymer.

Patent Literature 2 discloses an external preparation for skin comprising a composite nanoparticle comprising (A) hyaluronic acid and (B) a zwitterionic compound, wherein the particle diameter is 100nm or less.

[CITATION LIST]

[PATENT LITERATURE]

[PTL 1] Japanese Unexamined Patent Publication (Kokai) No. 2010-150151 [PTL 2] WO 2018/182003

SUMMARY

[TECHNICAL PROBLEM]

Since the stratum corneum located in the outermost layer of the skin has a barrier function to prevent foreign substances from entering the skin from the outside world, it has a property of hardly allowing the active ingredient applied to the skin to reach the inside of the skin. Therefore, when hyaluronic acid is simply applied to the skin, the moisturizing effect of hyaluronic acid tends to remain on the surface of the skin, and only a short-term moisturizing effect can be exhibited. As a result, it was necessary to continue to apply hyaluronic acid frequently and long-term when improving skin wrinkles and the like.

For example, when hyaluronic acid is injected into the inside of the skin using an injection, the persistence of the moisturizing effect is improved as compared with a case where it is applied, but this method has a problem that it is painful due to the invasiveness of an injection.

Particles on the order of nanometers, including hyaluronic acid, as described in PTL 1 and PTL 2, can penetrate deeper into the skin than the stratum corneum. However, since the content ratio of hyaluronic acid in the particles described in PTL 1 is as low as about 3% by mass, and the content ratio of hyaluronic acid in the particles described in PTL 2 is also 50% by mass or less, there has been a case where sufficient moisture retaining property cannot be secured.

Accordingly, it is a subject of the present disclosure to provide a cosmetic comprising a hyaluronic acid particle, which can maintain a non-invasively excellent moisture retaining property over a long period of time.

[SOLUTION TO PROBLEM]

<Aspect 1>

A cosmetic, comprising an aqueous medium, and a hyaluronic acid particle dispersed in the aqueous medium, wherein the average particle diameter of the hyaluronic acid particle is 200nm or less.

<Aspect 2>

The cosmetic according to aspect 1, wherein the weight average molecular weight of the hyaluronic acid is 10,000,000 or less.

<Aspect 3>

The cosmetic according to aspect 1 or 2, comprising at least one salt selected from an inorganic salt and an organic acid salt.

<Aspect 4>

The cosmetic according to aspect 3, wherein the ionic strength of the salt is 0.01 or more.

<Aspect 5>

The cosmetic according to any one of aspects 1 to 4, wherein the content of the hyaluronic acid particle is 0.005% by mass or more.

<Aspect 6>

The cosmetic according to any one of aspects 1 to 5, wherein the content of an ionic compound, glycols, ethanol, and urea is 15% by mass or less, respectively.

<Aspect 7>

The cosmetic according to any of aspects 1 to 6, which is applied to the skin.

<Aspect 8>

A method for producing a cosmetic according to any one of aspects 1 to 7, wherein after blending a salt into water or a buffer, hyaluronic acid is further blended to prepare a hyaluronic acid particle.

<Aspect 9>

A method for producing a cosmetic according to any one of aspects 1 to 7, wherein hyaluronic acid is blended into water or a buffer to dissolve hyaluronic acid, and then the salt is further blended to prepare a hyaluronic acid particle.

[ADVANTAGEOUS EFFECTS OF INVENTION]

According to the present disclosure, it is possible to provide a cosmetic comprising a hyaluronic acid particle, which can maintain a non-invasively excellent moisture retaining property over a long period of time.

BRIEF DESCRIPTION OF DRAWINGS

FIG. 1 is a graph related to Z average particle diameter and ionic strength of hyaluronic acid particles prepared using sodium chloride.
FIG. 2 is a graph related to Z average particle diameter and ionic strength of hyaluronic acid particles in a composition of each hyaluronic acid concentration prepared using sodium chloride.
FIG. 3 is a graph related to Z average particle diameter and ionic strength of hyaluronic acid particles in each composition having hyaluronic acid concentrations of 0.4% by mass or 0.5% by mass prepared using sodium chloride.
FIG. 4 is a graph related to Z average particle diameter and moisture retaining property of hyaluronic acid particles.
FIG. 5 is a graph related to Z average particle diameter and ionic strength of hyaluronic acid particles prepared using citrate buffer.
FIG. 6 is a graph related to Z average particle diameter of hyaluronic acid particles with addition of urea.

DESCRIPTION OF EMBODIMENTS

Hereinafter, embodiments of the present disclosure will be described in detail. The present disclosure is not limited to the following embodiments, and may be variously modified and practiced within the scope of the present invention.
The cosmetic of the present disclosure contains an aqueous medium and a hyaluronic acid particle dispersed in the aqueous medium, and the average particle diameter of the hyaluronic acid particle is 200nm or less.
Although not limited by the principle, it is considered that the action principle in which the cosmetic containing hyaluronic acid particles of the present disclosure can maintain a non-invasively excellent moisture retaining property over a long period of time, and the action principle in which hyaluronic acid particles having an average particle diameter of 200nm or less and highly containing a hyaluronic acid component can be prepared is as follows.

It is considered that hyaluronic acid particles with a diameter of 200 nm or less, particularly 100nm or less, tend to non-invasively penetrate into the interior of the skin through the stratum corneum or pores of the skin or the like, but particles of such size may not be able to penetrate any extent into the skin interior, and the amount of penetration of particles is limited to some extent.

For example, in the composite nanoparticles described in PTL 2, the content of hyaluronic acid in the particles is 50% by mass or less, whereas the hyaluronic acid particles of the present disclosure contain hyaluronic acid in the particles at a ratio of more than 50% by mass. As a result, for example, when the particle diameter of the composite nanoparticles described in PTL 2 and the particle diameter of the hyaluronic acid particles of the present disclosure are the same and the number of particles entering the inside of the skin is the same, it is considered that the hyaluronic acid particles of the present disclosure can improve the moisturizing effect and its persistence inside the skin because the content ratio of hyaluronic acid in the particles is larger.

In general, the skin exposed to dryness is deprived of moisture without knowing, and the moisture content on the surface of the skin is not maintained. When the moisture on the skin surface becomes insufficient, the moisturizing ingredients (natural moisturizing factors: Nature Moisturizing Factor (NMF)) produced by the skin itself cannot be successfully produced. As a result, since the barrier function and the moisturizing function on the surface of the skin deteriorate, and the skin tends to be damaged, it is considered that the skin loses moisture and causes wrinkles, skin irritation, and the like. It is also considered that the cosmetic of the present disclosure can highly penetrate the hyaluronic acid component which functions as a moisturizing agent into the interior of the skin and retain the amount of moisture in the vicinity of the surface inside the skin for a long period of time, so that it is also possible to improve skin troubles such as wrinkles.

Hyaluronic acid generally has a negative charge due to the presence of a carboxyl group. Due to the electrostatic repulsion based on this negative charge, the molecules of hyaluronic acid were easily spread in a filamentous form and tended to be difficult to be in the form of particles. Therefore, in order to prepare hyaluronic acid molecules into particles, particularly particles with a diameter of 200nm or less, a support material of the order of nanometers capable of supporting hyaluronic acid molecules was required as in PTL 1 and PTL 2, so that a limit was imposed on the content ratio of hyaluronic acid in the particles.

The present inventor has focused on this electrostatic repulsion which exhibits an action of spreading hyaluronic acid molecules in a filamentous form and a hydrogen bond based on a hydroxyl group or the like in hyaluronic acid which exhibits an attracting action, and has found that by utilizing an electrostatic shielding effect by an electrolyte such as sodium chloride, the negative charge of hyaluronic acid is apparently neutralized and the hydrogen bond is made dominant, the spreading of hyaluronic acid molecules is suppressed, and even a hyaluronic acid molecule alone can be made into nanometer-order particles. As a result, since the hyaluronic acid particles of the present disclosure can be prepared without using a support material as described above, it has become possible to theoretically make the content ratio of hyaluronic acid in the particles 100% by mass.

Note that, even when an electrolyte such as sodium chloride is present in an aqueous hyaluronic acid solution, if a component or the like which adversely affects the apparent neutralization of a negative charge or hydrogen bonding, such as urea or an ionic surfactant, which will be described later, is included, it may be difficult to obtain an effect of suppressing the spread of hyaluronic acid molecules and/or an effect of attracting hyaluronic acid molecules.

In addition, the hyaluronic acid particles of the present disclosure can retain the form of particles in a cosmetic without using a crosslinking agent. This is considered to be a result of hydrogen bonding which exhibits an attractive action on the hyaluronic acid molecule intramolecularly and/or intermolecularly.

<<Cosmetic containing hyaluronic acid particles>>

The cosmetic of the present disclosure contains an aqueous medium and hyaluronic acid particles dispersed in the aqueous medium.

<Hyaluronic acid particle>

It is known that the penetration of hyaluronic acid particles into the skin may vary depending on the individual difference, and may vary depending on the state of the stratum corneum, the number or size of the pores, and the like, but if the particle is a particle having an average particle diameter of 200nm or less, the particle can penetrate into most of the skin. From the viewpoint of penetration into the skin, ease of preparation of particles, and the like, the average particle diameter of the hyaluronic acid particles may be, for example, 150nm or less, 120nm or less, or 100nm or less, and may be 10nm or more, 30nm or more, or 50nm or more. Here, the average particle diameter means a Z average particle diameter of hyaluronic acid particles optically measured by a dynamic light scattering method when the particle shape of hyaluronic acid particles is assumed to be spherical. Such an average particle diameter can be measured using, for example, a zetasizer (manufactured by Malvern Panalytical Co., Ltd.), or a dynamic light scattering photometer DLS-8000 (manufactured by Otsuka Electronics Co., Ltd.). These measuring devices can be appropriately selected based on the overlap concentration of each hyaluronic acid. For example, a dynamic light scattering photometer DLS-8000 may be used if it is less than the overlap concentration, and a zetasizer may be used if it is greater than or equal to the overlap concentration. The overlap concentration can be calculated, for example, by a confocal fluorescence recovery after photobleaching method (Confocal-FRAP).

It is considered that the hyaluronic acid particles of the present disclosure exhibit a filamentous ball form in which molecules of hyaluronic acid are entangled and aggregated. Since such particles can be prepared without using the support material described above, the content ratio of hyaluronic acid in the particles can be theoretically 100% by mass. In other words, the hyaluronic acid particles of the present disclosure can be composed of a hyaluronic acid molecule alone as a polymer component.

However, the hyaluronic acid particle of the present disclosure may contain other polymer components other than hyaluronic acid within a range that does not cause a defect in the average particle diameter, moisture retaining property, and the like. The content ratio of the other polymer component may be, for example, 20% by mass or less, 10% by mass or less, 5% by mass or less, 3% by mass or less, or 1% by mass or less based on the total polymer amount contained in the hyaluronic acid particle. In other words, in the present disclosure, the "hyaluronic acid particle" is intended to include a particle highly containing a hyaluronic acid component as described above, and particles having a proportion of a hyaluronic acid component of 50% by mass or less as described in PTL 1 and PTL 2 are not encompassed. Here, the content ratio of hyaluronic acid in the hyaluronic acid particle can be measured using, for example, an ultraviolet-visible spectrophotometer (V-530, manufactured by JASCO Corporation, measurement wavelength: 270nm).

As the content of the hyaluronic acid particles in the cosmetic, for example, from the viewpoint of moisture retaining property, cost, and the like, the content can be 0.005% by mass or more, 0.01% by mass or more, 0.05% by mass or more, 0.10% by mass or more, 0.15% by mass or more, 0.20% by mass or more, or 0.25% by mass or more based on the total amount of the cosmetic, and can be 1.0% by mass or less, 0.80% by mass or less, 0.60% by mass or less, 0.50% by mass or less, or 0.45% by mass or less. As described above, since the cosmetic of the present disclosure contains hyaluronic acid particles having a high content ratio of hyaluronic acid and an average particle diameter of 200nm or less that can easily penetrate the skin non-invasively, it is possible to exhibit a sufficient moisturizing effect even if the content of hyaluronic acid particles in the cosmetic is relatively low.

(Hyaluronic acid)

There is no particular limitation on hyaluronic acid which can constitute hyaluronic acid particles. In general, hyaluronic acid means a linear polymer in which N-acetyl-D-glucosamine residues and D-glucuronic acid residues are alternately linked, and such hyaluronic acid can be obtained, for example, by isolation extraction from chicken crowns or other animal tissues or by fermentation using microorganisms such as Streptococcus species.

Hyaluronic acid may be a derivative thereof. As derivatives of hyaluronic acid, for example, hyaluronic acid metal salts such as sodium salt of hyaluronic acid, potassium salt of hyaluronic acid, magnesium salt of hyaluronic acid, calcium salt of hyaluronic acid, and aluminum salt of hyaluronic acid can be used. The hyaluronic acid derivatives obtained by etherification, esterification, amidation, acetylation, acetalization, or ketalization of hydroxyl group, carboxy group, etc. of hyaluronic acid can be used. Here, "hyaluronic acid" in the present disclosure may encompass the concept of hyaluronic acid and derivatives thereof.

There is no particular limitation on the weight average molecular weight of hyaluronic acid, and for example, it can be 10,000,000 or less. In general, it is considered that a relatively low molecular weight hyaluronic acid having a weight average molecular weight of less than 500 tends to enter the skin even if it is not in the form of microparticle. However, such a low molecular weight hyaluronic acid hardly stays inside the skin, and since the retention performance of moisture is inferior to that of hyaluronic acid having a high molecular weight, it may be difficult to sustain the moisturizing effect inside the skin over a long period of time. On the other hand, the hyaluronic acid particles of the present disclosure can be prepared using hyaluronic acid having a high molecular weight, and also, such hyaluronic acid can be highly contained in the particles, so that the obtained hyaluronic acid particles tend to stay inside the skin, and the moisturizing effect inside the skin can be sustained over a long period of time. From the viewpoint of the retention performance of moisture inside the skin and the ease of preparation of the particles, the weight average molecular weight of the hyaluronic acid may be, for example, 500 or more, 1,000 or more, 5,000 or more, 10,000 or more, 50,000 or more, 100,000 or more, 300,000 or more, 500,000 or more, 800,000 or more, or 1,000,000 or more, and may be 10,000,000 or less, 8,000,000 or less, 5,000,000 or less, 3,000,000 or less, 2,000,000 or less, or 1,500,000 or less. Here, the weight average molecular weight is intended to be a weight average molecular weight in terms of polystyrene in gel permeation chromatography measurement.

Hyaluronic acid may be used alone or in combination of two or more of hyaluronic acid and its derivatives. Also, the molecular weights of the hyaluronic acid and its derivatives used may be the same or different.

Commercially available hyaluronic acid may be used. Examples of commercially available hyaluronic acid include hyaluronic acid HA-LQ (manufactured by Kewpie Corporation), hyaluronic acid FCH (manufactured by Kikkoman Biochemical Company), and sodium biohyaluronate HA12N (manufactured by Shiseido Co., Ltd.).

<Aqueous media>

There is no particular limitation on the aqueous medium, and an aqueous medium used in cosmetics, quasi-drugs, and the like can be used. For example, ion-exchanged water, distilled water, ultrapure water, tap water, buffer, or the like can be used.

Examples of the buffer include citrate buffer, lactate buffer, phosphate buffer, acetate buffer, tartrate buffer, borate buffer, and Tris buffer. From the viewpoint of high buffer capacity, citrate buffer, lactate buffer and phosphate buffer are preferred, and citrate buffer is more preferred.

The pH of the buffer may be 7.0 or less, 6.8 or less, or 6.5 or less. The lower limit value of the pH of the buffer is not particularly limited, but is preferably 4.5 or more, 5.5 or more, or 6.0 or more, for example, from the viewpoint of irritation to the skin.

<Salt>

The cosmetic of the present disclosure may contain a salt in a cosmetic because a salt is used at the time of preparation of hyaluronic acid particles. As the salt which can be contained in the cosmetic, there is no particular limitation as long as it is a salt capable of apparently neutralizing the negative charge of hyaluronic acid. Examples of such a salt include at least one salt selected from an inorganic salt and an organic acid salt. When considering use as a cosmetic, among inorganic salts and organic acid salts, it is preferable to be a salt which hardly exerts an adverse effect on the skin. Here, the term "inorganic salt" means a salt composed solely of an inorganic component, and may be referred to as a salt composed of an ion generated from an inorganic acid and an inorganic base. In addition, "organic acid salt" means a salt formed by bonding an organic acid and a metal ion. Note that the salt is generally present in the form of an ion derived from a salt in a cosmetic. Thus, in the present disclosure, for example, "a cosmetic comprising a salt" is intended to include a salt in the form of such an ion. Also, ionic surfactants are not encompassed by "salts" of the present disclosure.

Examples of the inorganic salt include sodium nitrate, sodium sulfate, sodium chloride, potassium nitrate, potassium sulfate, potassium chloride, calcium nitrate, calcium sulfate, calcium chloride, magnesium nitrate, magnesium sulfate, magnesium chloride, aluminum nitrate, aluminum sulfate, and aluminum chloride. These salts may be used alone or in combination of two or more thereof.

Examples of the organic acid salt include citrate, acetate, lactate, tartrate, succinate, malate, glycolate, salicylate, and pyrrolidone carboxylate. Specific examples thereof include salts in which organic acids such as citric acid, acetic acid, lactic acid, tartaric acid, succinic acid, malic acid, glycolic acid, salicylic acid, and pyrrolidone carboxylic acid are bonded to metal ions such as sodium ions, potassium ions, calcium ions, magnesium ions, and aluminum ions. These salts may be used alone or in combination of two or more thereof.

The amount of salt to be blended in the cosmetic may be defined as the ionic strength of the salt. The ionic strength of the salt may be, for example, 0.01 or more, 0.03 or more, or 0.05 or more. Although there is no particular limitation on the upper limit value of the ionic strength, for example, it may be 4.0 or less, 3.0 or less, 2.0 or less, or 1.0 or less. Here, for example, when a cosmetic is prepared by adding a salt to a buffer, the ionic strength is calculated based on all salt components including a salt component contained in the buffer itself and a salt component separately added to the buffer.

<Optional components>

In the cosmetic of the present disclosure, various components can be appropriately blended within a range that does not affect the effect of the present invention. Examples of such components include skin nutrients, vitamins, water-soluble agents applicable to pharmaceuticals, quasi drugs, cosmetics, and the like, ultraviolet absorbers, antioxidants, preservatives, antioxidant aids, thickeners, pigments, dyes, colorants, fragrances, and the like. These optional components may be used alone or in combination of two or more thereof.

Hyaluronic acid particles of the present disclosure are prepared by apparently neutralizing the negative charge of hyaluronic acid, and predominantly acting on intramolecular or intermolecular hydrogen bonds of hyaluronic acid, as described above. Therefore, for example, an additive which inhibits the apparent neutralization of the negative charge of hyaluronic acid and/or hydrogen bonding of hyaluronic acid to make the average particle diameter of the hyaluronic acid particles more than 200nm, or an additive which binds with the negative charge of hyaluronic acid and/or hydrogen bonds with hyaluronic acid to make the average particle diameter of the hyaluronic acid particles more than 200nm may be included in the range of 15% by mass or less, 10% by mass or less, 5% by mass or less, 1% by mass or less, 0.5% by mass or less, or 0.1% by mass or less, respectively, based on the total amount of the cosmetic, but these additives are preferably not included.

Examples of such additives include ionic compounds such as ionic surfactants, glycols such as 1,3-butylene glycol, dipropylene glycol, and isoprene glycol, ethanol, and urea. In particular, surfactants or compounds that exhibit cationic properties may electrostatically bond with hyaluronic acid having a negative charge to increase the particle diameter, and glycols and ethanol may also hydrogen bond with hyaluronic acid to increase the particle diameter. Further, since urea has an action of breaking an important hydrogen bond for particle formation, it may increase the particle diameter.

<<Preparation method of a cosmetic>>

The cosmetic of the present disclosure can be prepared, for example, using the following method.

A solution is prepared by blending a salt into water or a buffer, and hyaluronic acid is blended into the solution, and hyaluronic acid is dissolved while stirring and mixing to form hyaluronic acid particles, thereby preparing a cosmetic.

Alternatively, after the hyaluronic acid is blended into water or a buffer and stirred and mixed to dissolve the hyaluronic acid, a salt is further blended to form hyaluronic acid particles, thereby preparing a cosmetic.

Note that, when the buffer itself exhibits the above-described predetermined ionic strength by the action of the salt contained in the buffer, the addition of the salt can be omitted.

In addition, regarding as hyaluronic acid, water, a buffer, optional components, and the like, which can be used in the method for preparing a cosmetic, various materials in the items of the cosmetic described above can be used. Here, when an optional component is blended, such an optional component may be blended before becoming hyaluronic acid particles, but it is preferable to blend the optional component after preparing the hyaluronic acid particles so as not to affect the particle formation.

The amount of the hyaluronic acid to be blended may be 0.005% by mass or more, 0.01% by mass or more, 0.05% by mass or more, 0.10% by mass or more, 0.15% by mass or more, 0.20% by mass or more, or 0.25% by mass or more based on the total amount of the cosmetic, and may be 1.0% by mass or less, 0.80% by mass or less, 0.60% by mass or less, 0.50% by mass or less, or 0.45% by mass or less.

<<Application site of a cosmetic>>

The cosmetic of the present disclosure can be applied and used anywhere on the surface of the skin in any portion of the body. For example, it can be suitably applied to a skin surface such as a face (lips, eyes, eyelids, cheeks, forehead, the space between the eyebrows, nose, or the like), ears, hands, arms, neck, legs, feet, chest, abdomen, back, or the like. Here, the skin also includes a nail or the like in which the corneum of the epidermis of the skin is changed and hardened.

<<Other applications of hyaluronic acid particles>>

In addition to the cosmetics, the hyaluronic acid particles of the present disclosure can also be used, for example, in applications such as an external preparation for skin, a medicine, and a quasi-drug.

EXAMPLES

Hereinafter, the present invention will be described in more detail with reference to Test Examples and Examples, but the present invention is not limited thereto.

<<Test Examples 1 to 6>>

<Evaluation of compositions>

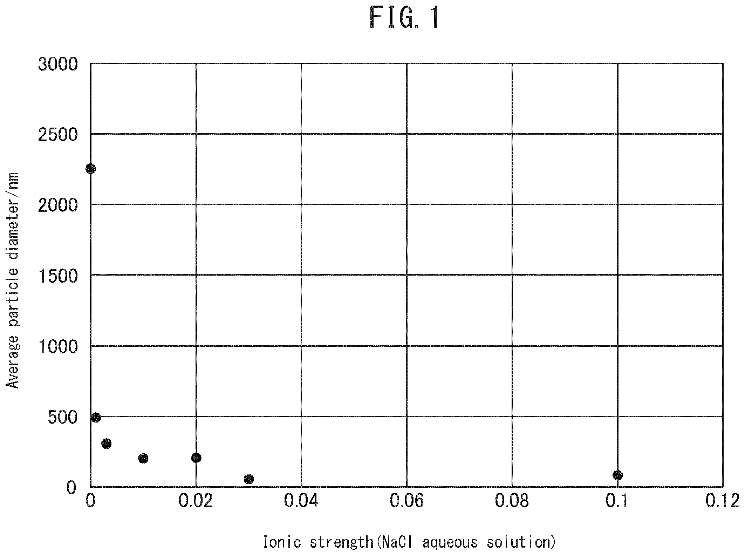

Various evaluations described below were performed on the compositions obtained by the following manufacturing methods, and the results are summarized in Tables 1 to 6 and Figures 1 to 6. Note that "HA" in the table and figures is intended to be hyaluronic acid.

(Evaluation of average particle diameter)

The average particle diameter of the hyaluronic acid particles in the composition was evaluated based on the Z average particle diameter by the dynamic light scattering method using a zetasizer (manufactured by Malvern Panalytical Co., Ltd.), or a dynamic light scattering photometer DLS-8000 (manufactured by Otsuka Electronics Co., Ltd.).

(Evaluation of moisture content ratio)

After washing the inner part of the upper arm of the subject with soap, the subject spent 20 minutes in a constant temperature and humidity room having a temperature of 21 $\pm$ 1 °C and a relative humidity of 45 $\pm$ 5% to adjust the environment of the subject. Then, the moisture content before applying the composition to the skin surface of the washed upper arm inner part and the moisture content at 20 minutes, 30 minutes, 60 minutes and 120 minutes after applying one drop ($2\times2cm^2$) of the composition to the skin surface were measured using a Corneometer ™ CM825(manufactured by Courage and Khazaka Co., Ltd.). From the obtained moisture content, the moisture content ratio was calculated by the following Formula 1:

$$\text{Moisture content ratio} = \text{Moisture content after application of the composition} / \text{Moisture content before application of the composition} \quad .. \text{Formula 1}$$

<Test Example 1: Effect of ionic strength>

In Test Example 1, the effect of ionic strength on the particle diameter of hyaluronic acid particles was studied. The results are shown in Table 1 and FIG. 1.

(Method for preparing a composition)

Hyaluronic acid (manufactured by Shiseido Co., Ltd., Biohyaluro 12: weight average molecular weight 1.2 million) was added to ion-exchanged water so as to have a content of 0.1 mass%, and the mixture was stirred and mixed by a vortex mixer to prepare Composition A. After dissolving the hyaluronic acid, sodium chloride was added at a concentration of 0.01M (mol/l), 0.003M, 0.01M, 0.02M, 0.03M, and 0.10M to each of the separated compositions A so that the ionic strength of the salt was 0.001, 0.003, 0.01, 0.02, 0.03, and 0.10, and the mixture was stirred and mixed by a vortex mixer to prepare each of the hyaluronic acid particles-containing compositions. Here, in the case of sodium chloride composed of a 1 valent cation and a 1 valent anion, the concentration of sodium chloride and the value of the ionic strength are the same.

[Table 1]

| HA concentration 0.1 mass% | Ionic strength | | | | | | |
|---|---|---|---|---|---|---|---|
| | 0 | 0.001 | 0.003 | 0.01 | 0.02 | 0.03 | 0.10 |
| Average particle diameter (nm) | 2,254 | 493 | 308 | 204 | 208 | 56 | 83 |

(Results)

As is apparent from the results of Table 1 and FIG. 1, it was confirmed that hyaluronic acid particles of the nanometer order were obtained by adding a salt. In addition, with an increase in the amount of salt added, that is, an increase in the ionic strength, it was confirmed that the particle diameter of the hyaluronic acid particles tended to decrease, and in particular, at an ionic strength of 0.03 or more, it was confirmed that hyaluronic acid particles with a diameter of 100nm or less could be obtained.

<Test Example 2: Effect of concentration of hyaluronic acid and ionic strength>

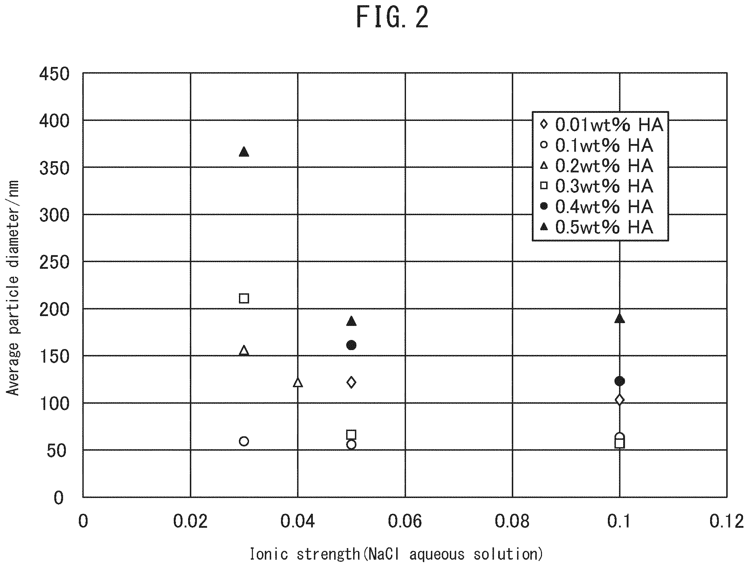

In Test Example 2, the effect of the concentration of hyaluronic acid and the ionic strength at the time of preparation of the composition on the particle diameter of the hyaluronic acid particles was studied. The results are shown in Table 2 and FIG. 2.

(Method for preparing a composition)

The hyaluronic acid particles-containing compositions were each prepared in the same manner as in Test Example 1, except that the concentration of hyaluronic acid at the time of preparation of the composition was 0.01 mass%, 0.1 mass%, 0.2 mass%, 0.3 mass%, 0.4 mass%, and 0.5 mass%, and sodium chloride was added so that the ionic strength was the ratio described in Table 2.

[Table 2]

| | HA concentration (mass%) | Ionic strength | | | | |
|---|---|---|---|---|---|---|
| | | 0.03 | 0.04 | 0.05 | 0.10 | 1.0 |
| Average particle diameter (nm) | 0.01 | - | - | 121 | 105 | 109 |
| | 0.1 | 59 | - | 56 | 64 | - |
| | 0.2 | 156 | 122 | - | - | - |
| | 0.3 | 211 | - | 66 | 57 | - |
| | 0.4 | - | - | 161 | 123 | - |
| | 0.5 | 367 | - | 187 | 190 | - |

(Results)

As is apparent from the results of Table 2 and FIG. 2, it was confirmed that even if the concentration of hyaluronic acid increased, hyaluronic acid particles of the nanometer order were obtained by adding a salt.

<Test Example 3: Effect of ionic strength when the concentration of hyaluronic acid is 0.4 to 0.5 mass%>

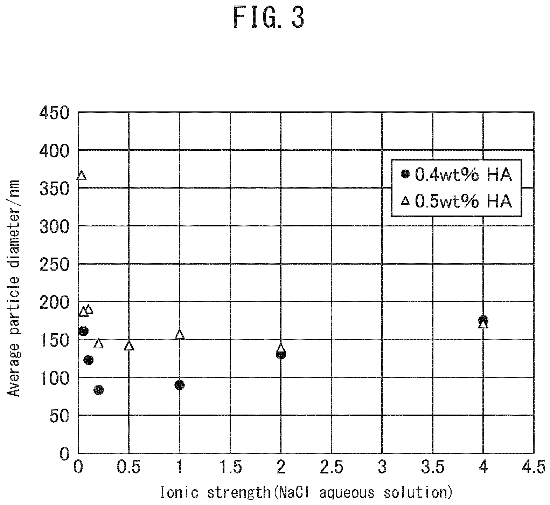

In Test Example 3, the effect of the ionic strength on the hyaluronic acid particles was studied when the concentration of hyaluronic acid at the time of preparation of the composition was 0.4 to 0.5 mass%. The results are shown in Table 3 and FIG. 3.

(Method for preparing a composition)

The hyaluronic acid particles-containing compositions were each prepared in the same manner as in Test Example 1, except that the concentration of hyaluronic acid was 0.4 mass% and 0.5 mass%, and sodium chloride was added so that the ionic strength was the ratio described in Table 3.

[Table 3]

| | HA concentration (mass%) | Ionic strength | | | | | | | |
|---|---|---|---|---|---|---|---|---|---|
| | | 0.03 | 0.05 | 0.10 | 0.20 | 0.50 | 1.0 | 2.0 | 4.0 |
| Average particle diameter (nm) | 0.4 | - | 161 | 123 | 84 | - | 90 | 130 | 175 |
| | 0.5 | 367 | 187 | 190 | 145 | 142 | 157 | 139 | 172 |

(Results)

As is apparent from the results of Table 3 and FIG. 3, it was confirmed that when the ionic strength was 0.05 or more, hyaluronic acid particles with a diameter of 200nm or less were obtained in both cases where the concentration of hyaluronic acid was 0.4 mass% and 0.5 mass%. In particular, when the concentration of hyaluronic acid was 0.4 mass%, it was confirmed that hyaluronic acid particles with a diameter of 100nm or less were obtained in the range of the ionic strength from 0.20 to 1.0.

<Test Example 4: Effect of particle diameter of hyaluronic acid particles on moisture retaining property>

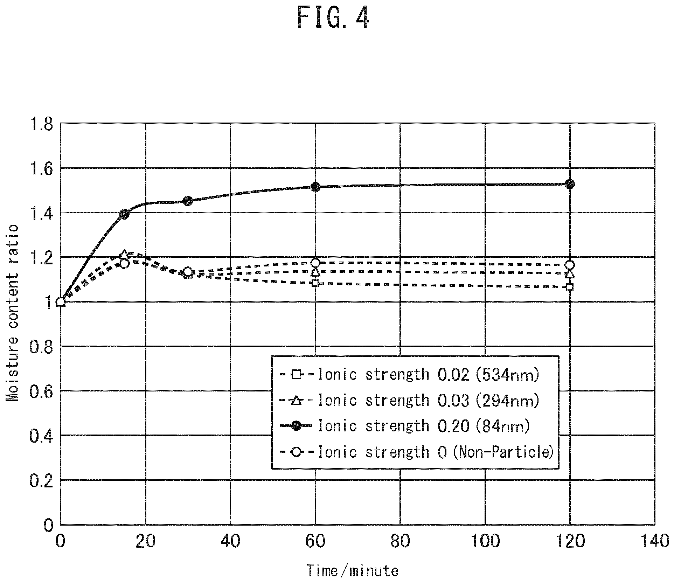

In Test Example 4, the effect of particle diameter of hyaluronic acid particles on moisture retaining property was studied. The results are shown in Table 4 and FIG. 4. Here, it can be said that as the moisture content ratio becomes larger than 1, the moisture retaining property is improved as compared with the state before the application of the composition. The moisture content ratio is preferably 1.25 or more, more preferably 1.30 or more, and particularly preferably 1.35 or more.

(Method for preparing a composition)

The hyaluronic acid particles-containing compositions were each prepared in the same manner as in Test Example 1, except that the concentration of hyaluronic acid was 0.4 mass%, and sodium chloride was added so that the ionic strength was the ratio described in Table 4. Here, since it is considered that the hyaluronic acid molecule in the composition having an ionic strength of 0 without adding sodium chloride spreads in a filamentous form and does not take the form of particles, it is denoted as "non-particle" in the table and the figure.

[Table 4]

| HA concentration 0.4 mass% | Ionic strength | Average particle diameter (nm) | Time (minute) | | | | |
|---|---|---|---|---|---|---|---|
| | | | 0 | 15 | 30 | 60 | 120 |
| Moisture content ratio | 0 | Non-Particle | 1.00 | 1.17 | 1.13 | 1.17 | 1.16 |
| | 0.02 | 534 | 1.00 | 1.18 | 1.12 | 1.08 | 1.07 |
| | 0.03 | 294 | 1.00 | 1.21 | 1.13 | 1.14 | 1.13 |
| | 0.20 | 84 | 1.00 | 1.39 | 1.45 | 1.51 | 1.53 |

(Results)

As is apparent from the results of Table 4 and FIG. 4, in the case of a composition containing hyaluronic acid that is not in the form of particle and a composition containing hyaluronic acid particles having an average particle diameter of 294nm or more, immediately after the composition was applied to the skin, the moisture retaining property temporarily increased, but the moisture retaining property thereof was lowered in only about 30 minutes, and exhibited only almost the same degree of moisture retaining property as in a state where the composition was not applied. It is considered that this is because the hyaluronic acid in these compositions has not penetrated into the skin interior.
On the other hand, in the case of a composition containing hyaluronic acid particles having an average particle diameter of 84nm, it was confirmed that an excellent moisture retaining property was exhibited as soon as the composition was applied to the skin, and that its moisture retaining property could be maintained over a long period of time. It is considered that the action effect on the immediate effect and persistence of this moisture retaining property is caused by the fact that the hyaluronic acid particles are in an ultrafine particle form of 200nm or less which is easy to penetrate into the inside of the skin and the high content ratio of hyaluronic acid in the particles.

<Test Example 5: Effect of particle diameter of hyaluronic acid particles by sodium citrate>

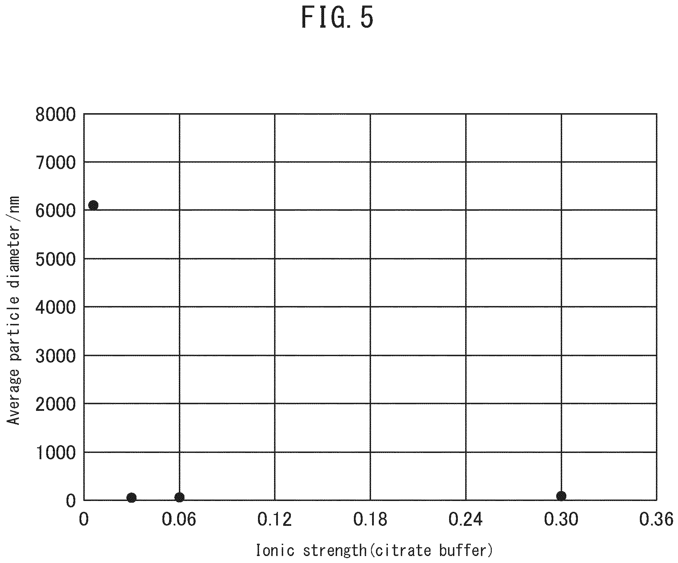

In Test Example 5, the effect of the particle diameter of hyaluronic acid particles by sodium citrate was studied. The results are shown in Table 5 and FIG 5.

(Method for preparing a composition)

Hyaluronic acid (manufactured by Shiseido Co., Ltd., Biohyaluro 12: weight average molecular weight 1.2 million) was added to a citrate buffer (manufactured by Fujifilm Wako Pure Chemical Co., Ltd.: pH 6.5, ionic strength 0.006) so as to have a content of 0.1 mass%, and the mixture was stirred and mixed by a vortex mixer to prepare Composition B. After dissolving the hyaluronic acid, sodium citrate was added to each of the separated compositions B so that the ionic strength of the salt was 0.03, 0.06, and 0.30, and the mixture was stirred and mixed by a vortex mixer to prepare each of the hyaluronic acid particles-containing compositions. Here, the concentration of sodium citrate when the ionic strength was 0.03 was 0.005M, the concentration of sodium citrate when the ionic strength was 0.06 was 0.010M, and the concentration of sodium citrate when the ionic strength was 0.30 was 0.050M.

[Table 5]

| HA concentration 0.1 mass% | Ionic strength | | | |
|---|---|---|---|---|
| | 0.006 | 0.03 | 0.06 | 0.30 |
| Average particle diameter (nm) | 6,100 | 51 | 60 | 86 |

(Results)

As is apparent from the results of Table 5 and FIG. 5, it was confirmed that hyaluronic acid particles with a diameter of 200nm or less were obtained even in salts other than sodium chloride.

<Test Example 6: Effect of particle diameter of hyaluronic acid particles with use of additives inhibiting hydrogen bonding>

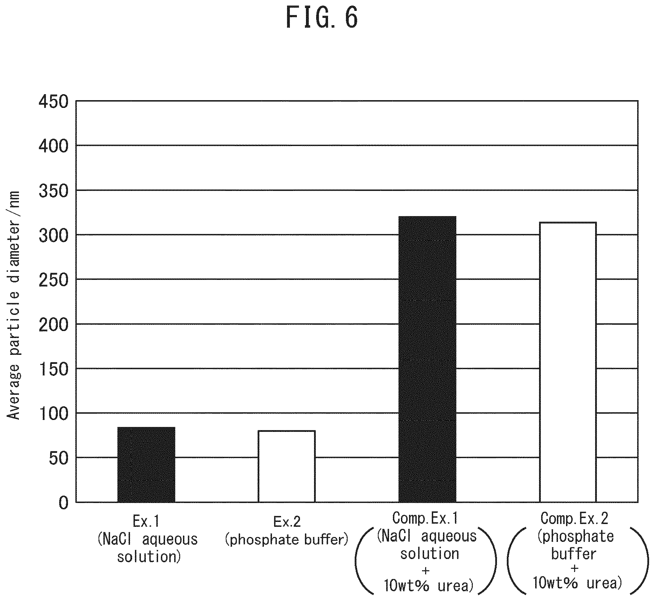

From Test Example 6, the effect of the particle diameter of hyaluronic acid particles accompanying the use of urea which is an additive that inhibits hydrogen bonding was studied. The results are shown in Table 6 and FIG. 6.

(Example 1)

Hyaluronic acid (manufactured by Shiseido Co., Ltd., Biohyaluro 12: weight average molecular weight 1.2 million) was added to ion-exchanged water so as to have a content of 0.1 mass%, and the mixture was stirred and mixed by a vortex mixer to prepare Composition C. After dissolving the hyaluronic acid, sodium chloride was added at a concentration of 0.10M to composition C so that the ionic strength of the salt was 0.10, and the mixture was stirred and mixed by a vortex mixer to prepare the hyaluronic acid particles-containing composition.

(Example 2)

Hyaluronic acid (manufactured by Shiseido Co., Ltd., Biohyaluro 12: weight average molecular weight 1.2 million) was added to an isotonic phosphate buffer (manufactured by Takara Bio Inc.: pH 7.4, ionic strength 0.154) so as to have a content of 0.1 mass%, and the mixture was stirred and mixed by a vortex mixer to prepare the hyaluronic acid particles-containing composition.

(Comparative Example 1)

The composition of Comparative Example 1 was prepared by blending 10 mass% of urea with respect to the total amount of the hyaluronic acid particles-containing composition of Example 1.

(Comparative Example 2)

The composition of Comparative Example 2 was prepared by blending 10 mass% of urea with respect to the total amount of the hyaluronic acid particles-containing composition of Example 2.

[Table 6]

| | Ex. 1 | Ex. 2 | Comp. Ex. 1 | Comp. Ex. 2 |
|---|---|---|---|---|
| Average particle diameter (nm) | 83 | 80 | 320 | 314 |

(Results)

From the results of Table 6 and FIG. 6, it was confirmed that when urea which exhibits an action of breaking hydrogen bonds was added into the composition, the particle diameter of the hyaluronic acid particles increased greatly. As is also apparent from this result, it is considered that the hyaluronic acid particles prepared by the method of the present disclosure are not particles as obtained by crosslinking bonding not affected by urea, but particles which are formed by hydrogen bonding, since the particle diameter is varied by the formulation of urea which breaks hydrogen bonds.

<<Example of cosmetic formulation>>

Hereinafter, examples of formulation when hyaluronic acid particles prepared by the method of the present disclosure are used as cosmetics will be described, but the present invention is not limited to this illustration. When the cosmetic described in the following formulation example was applied to the skin, it was possible to exhibit excellent moisture retaining property over a long period of time.

<Formula Example 1: Cosmetic>

| (Component) | (mass%) |
|---|---|
| Ion exchange water | residue |
| Hyaluronic acid | 0.1 |
| Sodium chloride | 0.1 |
| Fragrance | proper quantity |

(Method for producing a cosmetic)

Hyaluronic acid was added to ion-exchanged water, and the mixture was stirred and mixed by a vortex mixer to dissolve hyaluronic acid. Then, sodium chloride was added to the prepared aqueous hyaluronic acid solution, and the mixture was stirred and mixed by a vortex mixer to prepare hyaluronic acid particles, and then fragrance was added to prepare a cosmetic.

**Claims**

1. A cosmetic, comprising an aqueous medium, and a hyaluronic acid particle dispersed in the aqueous medium, wherein the average particle diameter of the hyaluronic acid particle is 200nm or less.

2. The cosmetic according to claim 1, wherein the weight average molecular weight of the hyaluronic acid is 10,000,000 or less.

3. The cosmetic according to claim 1 or 2, comprising at least one salt selected from an inorganic salt and an organic acid salt.

4. The cosmetic according to claim 3, wherein the ionic strength of the salt is 0.01 or more.

5. The cosmetic according to any one of claims 1 to 4, wherein the content of the hyaluronic acid particle is 0.005% by mass or more.

6. The cosmetic according to any one of claims 1 to 5, wherein the content of an ionic compound, glycols, ethanol, and urea is 15% by mass or less, respectively.

7. The cosmetic according to any of claims 1 to 6, which is applied to the skin.

8. A method for producing a cosmetic according to any one of claims 1 to 7, wherein after blending a salt into water or a buffer, hyaluronic acid is further blended to prepare a hyaluronic acid particle.

9. A method for producing a cosmetic according to any one of claims 1 to 7, wherein hyaluronic acid is blended into water or a buffer to dissolve hyaluronic acid, and then the salt is further blended to prepare a hyaluronic acid particle.

# FIG. 1

FIG. 2

EP 4 018 998 A1

# FIG. 3

# FIG. 4

# FIG. 5

# FIG. 6

## INTERNATIONAL SEARCH REPORT

International application No.

PCT/JP2020/031318

### A. CLASSIFICATION OF SUBJECT MATTER

A61K 8/73(2006.01)i; A61K 8/04(2006.01)i; A61Q 19/00(2006.01)i
FI: A61K8/73; A61K8/04; A61Q19/00

According to International Patent Classification (IPC) or to both national classification and IPC

### B. FIELDS SEARCHED

Minimum documentation searched (classification system followed by classification symbols)
A61K8/73; A61K8/04; A61Q19/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | ZHU, Ye et al., "Self-assembly and emulsification of dopamine-modified hyaluronan", Carbohydrate Polymers, 2015, vol. 123, pp. 72-79, in particular, abstract, page 74, right column, page 78, left column, fig. 1, 2, 7 | 1-9 |
| X | JP 2014-076971 A (POLA CHEMICAL INDUSTRIES, INC.) 01.05.2014 (2014-05-01) claims, paragraphs [0013], [0017], [0021], [0031]-[0036], [0049] | 1-9 |
| X | JP 2012-506900 A (UNIVERSIDADE DE SANTIAGO DE COMPOSTELA) 22.03.2012 (2012-03-22) claims, paragraphs [0035], [0043], [0063], [0086], [0139] | 1-2, 5-7 |
| A | JP 2017-066046 A (TOKUTOME, Yoshihiro) 06.04.2017 (2017-04-06) claims, paragraphs [0017], [0022], [0039], [0048], [0053] | 1-2, 5-7 |
| A | US 2015/0080333 A1 (UNIVERSITY OF KANSAS) 19.03.2015 (2015-03-19) entire text, all drawings | 1-9 |

☒ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 October 2020 (06.10.2020) | 20 October 2020 (20.10.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/031318

C (Continuation). DOCUMENTS CONSIDERED TO BE RELEVANT

| Category* | Citation of document, with indication, where appropriate, of the relevant passages | Relevant to claim No. |
|---|---|---|
| A | US 2008/0160096 A1 (BERBELY, Janos et al.) 03.07.2008 (2008-07-03) entire text, all drawings | 1-9 |
| A | HAN, Song-Yi et al., "Mineralized hyaluronic acid nanoparticles as a robust drug carrier", Journal of Materials Chemistry, 2011, vol. 21, pp. 7996-8001 entire text, all drawings | 1-9 |

Form PCT/ISA/210 (continuation of second sheet) (January 2015)

| INTERNATIONAL SEARCH REPORT | | International application No. |
|---|---|---|
| Information on patent family members | | PCT/JP2020/031318 |

| Patent Documents referred in the Report | Publication Date | Patent Family | Publication Date |
|---|---|---|---|
| JP 2014-076971 A | 01 May 2014 | (Family: none) | |
| JP 2012-506900 A | 22 Mar. 2012 | US 2011/0256059 A1 claims, paragraphs [0052], [0060], [0085], [0108], [0162] WO 2010/049562 A1 EP 2366386 A1 | |
| JP 2017-066046 A | 06 Apr. 2017 | (Family: none) | |
| US 2015/0080333 A1 | 19 Mar. 2015 | WO 2013/109959 A1 | |
| US 2008/0160096 A1 | 03 Jul. 2008 | (Family: none) | |

Form PCT/ISA/210 (patent family annex) (January 2015)

**REFERENCES CITED IN THE DESCRIPTION**

**Patent documents cited in the description**

- JP 2010150151 A

- WO 2018182003 A